# EUROPEAN PATENT APPLICATION

(11) **EP 3 228 318 A1**
(43) Date of publication of application: **11.10.2017**
(21) Application number: 17164465.1
(22) Date of filing: 03.04.2017
(51) Int. Cl.: A61K 36/82, A61K 35/02, A61K 35/742, A61P 31/04, A61P 31/12

(54) **COMPOSITION COMPRISING DIOSMECTITE**

(30) Priority: 04.04.2016 IT UA20162277
(71) Applicant: Chemist's Research S.r.l., 73020 Cavallino (IT)
(72) Inventor: DEGLI ATTI, Arnaldo, 73100 LECCE (IT)
(74) Representative: Cattaneo, Elisabetta

(57) **Abstract**

The invention concerns a composition comprising diosmectite and decaffeinated green tea extract. Preferably, said composition comprises also Bacillus clausii spores, and it is preferably in the form of a powder. The composition has good palatability, it is easy to prepare and it resulted to be effective in the treatment of viral and bacterial gastrointestinal disorders, including intestinal flu, food poisoning, also deriving from contaminated seafood, and traveller's diarrhea.

## Description

### FIELD OF THE INVENTION

The present invention relates to a composition comprising diosmectite and decaffeinated green tea extract. In a preferred embodiment, the composition also comprises spores of the Bacillus clausii specie. The composition is extremely stable and has excellent palatability, while being safe and effective in treating gastrointestinal disorders.

### BACKGROUND

Diosmectite is a type of smectite clay made up of aluminum and magnesium silicates. It is a fully inorganic mixture of compounds, characterized by a complex chemical composition, but with well-defined morphological and crystallographic characteristics. The commercial term "diosmectite" refers to the dioctahedral smectite, to describe its peculiar crystallographic structure. Dioctahedral smectite, also simply smectite, finds various industrial applications. In the pharmaceutical field, its use in the therapeutic treatment of acute gastroenteritis and diarrhea symptoms is consolidated. In particular, it is known its use as an ingredient in products for the management of various clinical pictures in the gastrointestinal field, such as diarrhea and food allergies. In acute diarrhea in pediatric age, beneficial effects in terms of reduction of diarrhea duration, frequency of liquid stools and number of cases of prolonged diarrhea have been disclosed. The simplest description of the mechanism of action of this product reported in the literature considers the affinity for water of this clay, which, although completely insoluble, is capable of absorbing water up to eight times its weight, converting into a high viscosity mass, and thus slowing down the pathological expulsion process. Although its use in the treatment of gastroenteritis is known, due to its peculiar taste, it has an unpleasant palatability for certain patients, especially in pediatric age.

In US2010/0159013 a therapeutic composition, flavored with traditional flavors, containing clay, in the form of a dioctahedral smectite is described, wherein the added flavor is encapsulated in a sugar-based encapsulating matrix, or derivatives thereof, obtained by extrusion techniques. Specifically, the encapsulated flavor is obtained by a preparation process which involves mixing the flavor together with the sugar, or a derivative thereof, warming the obtained mixture to remove water, then adding an emulsifier under stirring to promote the homogenization of the molten mass. The molten mixture thus obtained is then extruded under pressure in a cold solvent, in order to crush the extrudate. Such an extrudate, consisting of the encapsulated flavor, is then added to the clay in order to flavor it, for obtaining products which comprise it, more pleasant to the intake. The only present example describes a clay mixed simultaneously with three different flavors (orange, caramel and vanilla) in order to obtain a composition with satisfactory palatability. Although the product has a certain organoleptic quality, the preparation process is clearly rather long and complex.

The object of the present invention is therefore to provide a diosmectite product having good organoleptic properties, but being at the same time easy to prepare.

### SUMMARY OF THE INVENTION

The inventors of the present invention have surprisingly found another active ingredient, in addition to diosmectite, that associated with diosmectite allows to obtain a product with excellent palatability through a simple mixing process, while obtaining a safe and effective product for the treatment of gastrointestinal diseases.

Therefore, the invention concerns a composition comprising diosmectite and decaffeinated green tea extract. Specifically, the composition of the invention comprises decaffeinated green tea, *i.e.* a green tea extract titrated at 98% in polyphenols, whose at least 40% is epigallocatechin gallate (EGCG). In a preferred embodiment, the invention concerns diosmectite, decaffeinated green tea extract, and Bacillus clausii spores.

The inventors of the present invention have, therefore, surprisingly found that by specifically placing the decaffeinated green tea extract together with diosmectite, a pleasant composition with good palatability was obtained.

In an advantageous embodiment of the invention, the composition is in the form of a powder formulation, preferably for oral use.

In another aspect, the invention concerns the composition or formulation for use as a medicament.

The composition of the invention therefore is an advanced formulation comprising, in addition to diosmectite, functional substances, such as polyphenols, whose at least 40% is epigallocatechin gallate, contained in the decaffeinated green tea extract and, preferably, Bacillus clausii spores, which allow, thanks to their mechanisms of action, to restore the physiological gastrointestinal function, while treating the gastrointestinal disorder and relative symptoms thereof.

In another aspect, therefore, the invention relates to the composition or formulation of the invention for use in the treatment of viral and bacterial gastrointestinal disorders. Among the disorders, the composition is effective in treating intestinal flu, food poisoning, also deriving from contaminated seafood, and traveller's diarrhea.

### DETAILED DESCRIPTION SUMMARY OF THE INVENTION

Therefore, the invention concerns a composition comprising diosmectite and decaffeinated green tea extract.

The preferably used diosmectite, *i.e.* clay defined as dioctahedral smectite, is a commercially available diosmectite, whose X-rays show smectite, as main component, with small traces of kaolinite.

Preferably, the composition comprises from 40 to 60% by weight of diosmectite, based on the total weight of the composition, preferably about 58%, even more preferably 58,42%, based on the total weight of the composition.

The composition of the invention also comprises decaffeinated green tea extract, *i.e.* a green tea extract titrated at 98% in polyphenols, whose at least 40% is epigallocatechin gallate (EGCG).

Advantageously, the decaffeinated green tea extract also confers astringent characteristics to the final composition, particularly useful in the treatment of the intestinal mucosa in case of traveller's diarrhea.

The inventors of the present invention have surprisingly found that, by specifically placing the decaffeinated green tea extract together with diosmectite, a pleasant composition of good palatability was obtained.

Preferably, the decaffeinated green tea extract is a decaffeinated green tea extract titrated at 98% in polyphenols, whose at least 40% is epigallocatechin gallate

(EGCG). Preferably the composition comprises from 1 to 3% by weight of decaffeinated green tea extract, based on the total weight of the composition, even more preferably about 1.95%, based on the total weight of the composition.

The composition of the invention may also comprise Bacillus clausii spores. It is known that Bacillus clausii may be used as a probiotic, in particular it is known that the enzymes produced by the metabolic activity of Bacillus clausii act in the intestine by improving the microbial balance of the human microbiota, and protecting it from other bacterial infections. For example, several clinical trials demonstrated the efficacy and tolerability of Bacillus clausii spores in the event of alterations (dysbiosis) of human microbiota, meteorism, diarrhea, and abdominal pain. The spores of Bacillus clausii are, in fact, currently used in probiotic drugs primarily indicated for the prevention and treatment of intestinal dismicrobism, both in adult patients and in pediatric patients.

In a preferred form, the composition of the invention therefore comprises Bacillus clausii spores in the range from 0.5 to 2% by weight of Bacillus clausii spores, based on the total weight of the composition, preferably about 1.1 %, based on the total weight of the composition.

In all embodiments, the composition of the invention was found to be palatable and pleasant to taste, without however requiring a complex preparation process, because the better palatability is due to the concomitant presence of the active ingredients diosmectite and decaffeinated green tea extract.

In a preferred embodiment of the invention, the composition of the invention is in the form of powders.

The composition of the invention also includes excipients for the preparation of a final formulation to be administered.

Preferably, said final formulation comprises suitable excipients for oral administration, preferably in the form of tablets, capsules, powders, granules, syrups, gels, aqueous suspensions, liquids.

Preferably, said excipients are selected from the group consisting of sweeteners, diluents, bulking agents, acidifying agents, and flavors.

Among sweeteners, aspartame, saccharin, sorbitol, mannitol, sucralose, acesulfame K, steviol glycosides, and mixtures thereof, can be mentioned.

Among diluents, maltodextrins can be mentioned.

Among bulking agents, calcium phosphate and microcrystalline cellulose can be mentioned.

As acidifying agent, citric acid, tartaric acid, malic acid, or mixtures thereof, may be used.

In a preferred embodiment, the formulation comprising the composition of the invention is in the form of powders.

In a further preferred embodiment, the formulation comprising the composition of the invention is in the form of granules.

In a further preferred embodiment, the formulation comprising the composition of the invention dispersed in water is in the form of a suspension.

In yet another preferred embodiment, the formulation comprising the composition of the invention dispersed in a suitable vehicle is in the form of a gel. Advantageously, said formulation comprising the composition of the invention, in the form of powder, granule, suspension, or gel, is provided in the form of a sachet, vial or stick.

Said formulation comprising the composition of the invention is preferably for oral administration.

Advantageously, the formulation comprising the composition of the invention, in the form of powder, granule, suspension or gel, provided in the form of a sachet, vial or stick, is in the form of a single-dose for oral administration.

Preferably, the formulation comprising the composition of the invention is in the form of powders, provided in the form of a single-dose sachet for oral administration.

The formulation comprising the composition of the invention, preferably in the form of a powder, even more preferably diluted in water, once taken, reaches the gastrointestinal mucosa where diosmectite, such as natural silicate of aluminum and magnesium, acts as an adsorbent against the toxic substances and toxins, and the decaffeinated green tea extract, rich in polyphenols and tannins, exerts an astringent, protective and consequently anti-inflammatory action towards the mucosa itself.

Said formulation comprising the composition of the invention, in a preferred embodiment also includes Bacillus Clausii, which, being not normally resident in the intestinal mucosa, acts as a probiotic, re-equilibrating also the intestinal bacterial flora, and thus promoting the reconstitution of the intestinal mucosa integrity.

Thus, the formulation comprising the composition of the invention, through the above described mechanism of action, helps to restore the physiological function of the gastrointestinal mucosa and to reduce the symptoms associated with gastrointestinal disorders.

In another aspect, the invention concerns to the composition or formulation for use as a medicament.

In another aspect, therefore, the invention concerns to the composition or formulation of the invention for use in the treatment of gastrointestinal disorders of viral and bacterial origin. Among disorders, the composition or formulation is suitable to effectively treat intestinal flu, food poisoning, also deriving from contaminated seafood, and traveller's diarrhea.

The composition or formulation for use of the invention preferably comprises unitary doses of the various active ingredients.

Preferably, the composition or formulation for use comprises a unitary dose of diosmectite from 1 to 4 g, preferably 3 g.

Preferably, the composition or formulation for use comprises a unitary dose of decaffeinated green tea extract from 70 to 200 mg, more preferably 100 mg.

Preferably, the composition or formulation for use also includes a unitary dose of Bacillus clausii from 0.01 to 1 g, more preferably 0.055 g. More preferably, the composition or formulation for use comprises a unitary dose of Bacillus clausii equal to about 5 Billions of Bacillus clausii (100MLD).

In a preferred embodiment, the composition of the invention is a powder formulation for the treatment of gastrointestinal disorders of viral and bacterial origin, preferably prepared as unitary dose sachets for oral administration. Advantageously, the composition of the invention is a powder formulation for the treatment of gastrointestinal disorders of viral and bacterial origin, preferably prepared as a unitary dose sachet for oral administration, comprising from 1 to 4 g of diosmectite and from 70 to 200 mg of decaffeinated green tea extract.

More preferably, said composition of the invention is a powder formulation for the treatment of gastrointestinal disorders of viral and bacterial origin, preferably prepared as a unitary dose sachet for oral administration, comprising from 1 to 4 g of diosmectite, from 70 to 200 mg of decaffeinated green tea extract, and from 0.01 to 1 g of Bacillus clausii.

Even more preferably, said composition of the invention is a powder formulation for the treatment of gastrointestinal disorders of viral and bacterial origin, prepared as a unitary dose sachet for oral administration, comprising 3 g of diosmectite, 0.055 g of Bacillus clausii, and 100 mg of decaffeinated green tea extract. Therefore, the composition or formulation of the invention, comprising diosmectite and decaffeinated green tea extract, and preferably spore of Bacillus clausii, resulted to be surprisingly palatable and pleasant to taste, as will be clear from the detailed description below. Furthermore, the composition or formulation of the invention resulted to be safe and effective in the treatment of gastrointestinal disorders of viral and bacterial origin.

### Experimental Part

### Example 1

### Preparation of Composition A of the Invention

For the preparation of composition A of the invention, the following ingredients in powder were employed.

| **COMPONENTS** | **% in g** | **g/sachet (5.13600 g)** |
|---|---|---|
| Diosmectite | 58.42 | 3.00000 |
| Decaffeinated Green Tea Extract (Titrated at 98% in polyphenols, whose at least 40% is epigallocatechin gallate (EGCG)) | 1.95 | 0.10000 |
| Bacillus Clausii 100 MLD | 1.1 | 0.05500 |
| Sucralose (Sweetener) | 0.151 | 0.00775 |
| Acesulfame(Sweetener) | 0.049 | 0.00250 |
| Maltodextrins (Diluent) | 35.81 | 1.839 |
| Citric Acid (Acidifying agent) | 1.46 | 0,075 |
| Tea Spray Flavor | 0.88 | 0.045 |
| Lemon Spray Flavor | 0.22 | 0.01125 |

The ingredients of the composition, sieved and weighed, in the quantities and in the order indicated in the table, were introduced into the mixer, and the final powder mixture thus obtained was left in the mixer under stirring for 15 minutes.

After this mixing step to homogenize the ingredients, the powder composition is ready for use and, in particular, it can be packaged.

### Example 2

### Preparation of Composition B of the Invention

For the preparation of composition B of the invention, the following ingredients in powder were employed.

| **COMPONENTS** | **% in g** | **g/sachet (5.13600 g)** |
|---|---|---|
| Diosmectite | 58.42 | 3.00000 |
| Decaffeinated Green Tea Extract (Titrated at 98% in polyphenols, whose at least 40% is epigallocatechin gallate (EGCG)) | 1.95 | 0.10000 |
| Sucralose (Sweetener) | 0.151 | 0.00775 |
| Acesulfame(Sweetener) | 0.049 | 0.00250 |
| Maltodextrins (Diluent) | 36.87 | 1.894 |
| Citric Acid (Acidifying agent) | 1.46 | 0,075 |
| Tea Spray Flavor | 0.88 | 0.045 |
| Lemon Spray Flavor | 0.22 | 0.01125 |

The ingredients of the composition, sieved and weighed, in the quantities and in the order indicated in the table, were introduced into the mixer, and the final powder mixture thus obtained was left in the mixer under stirring for 15 minutes.

After this mixing step to homogenize the ingredients, the powder composition is ready for use and, in particular, it can be packaged.

### Example 3

**Assessment of the palatability of compositions A and B of the invention**

Composition A of Example 1 was dissolved in about 300 ml of water and subjected to assay by 10 adult subjects.

These subjects were asked to assess, on a scale from 1 to 5, (where 1 is "unpleasant taste" and 5 is "pleasant taste") the pleasantness of the suspension. The assessment provided the following evaluation:

| Subj. 1 | Subj. 2 | Subj. 3 | Subj. 4 | Subj. 5 | Subj. 6 | Subj. 7 | Subj. 8 | Subj. 9 | Subj. 10 |
|---|---|---|---|---|---|---|---|---|---|
| 4 | 4 | 5 | 5 | 4 | 5 | 5 | 3 | 5 | 5 |

The average rating resulted to be 4.5.

To the same subjects were then asked, after a suitable waiting time and after administration of about 200 ml of water, to assess the pleasantness of a suspension obtained with the commercial product Diosmectal comprising, *inter alia*, 3 g of diosmectite in addition to various sweeteners and flavors.

This second test provided the following evaluation:

| Subj. 1 | Subj. 2 | Subj. 3 | Subj. 4 | Subj. 5 | Subj. 6 | Subj. 7 | Subj. 8 | Subj. 9 | Subj. 10 |
|---|---|---|---|---|---|---|---|---|---|
| 2 | 5 | 3 | 3 | 4 | 3 | 4 | 5 | 3 | 3 |

The average rating resulted to be 3.5.

In the conducted test, the suspension obtained with composition A of the invention thus exhibited an excellent palatability, even greater than the palatability of the commercial product Diosmectal.

The obtained suspension was found to be highly appreciated also in terms of olfactory profile.

The inventors also tested, following an identical procedure, composition B of Example 2, which did not contain Bacillus clausii, and found that the palatability obtained an average score of about 3.9. Thus, also the specific composition B had been judged pleasant in terms of taste profile.

### Example 4

### Acute toxicity assessment of the composition of the invention

A biological study on the composition of the invention was carried out in order to provide the required data to evaluate the toxicity of the composition after oral administration. The assessment was carried out according to OECD No. 420.

During the study, composition A as prepared in Example 1 was administered at a dose of 2,000 mg/Kg to a group of 5 female rats through a gastroesophageal catheter. Four hours after the treatment, animals were fed with a standard diet. In order to carry out the study, the animals were monitored for 14 days to assess any signs of possible toxic symptoms, including:
- mortality;
- assessment of bodily organs function;
- assessment of mucosal conditions;
- assessment of somatomotor activity and sensory conditions.

The body weight was also weekly monitored.

At the end of the study an autopsy was performed on all animals.

The results obtained can be summarized as follows:
- Mortality: No cases were observed during the entire study;
- Clinical Symptoms: No abnormalities were observed in any animals;
- Body Growth: Body growth was in accordance with the standards of the species and breed;
- Autopsy: No abnormalities were observed in treated animals.

Based on the above results, that were interpreted according to OECD No. 420 dated December 17, 2001, the composition of the invention showed an LD₅₀ greater than 2,000 mg/Kg.

### Example 5

### Evaluation of delayed hypersensitivity of composition of the invention

A biological evaluation was conducted aimed to identifying the sensitizing effects of composition A of the invention using the delayed hypersensitivity test (GPMT test) according to ISO10993-10:2010. 15 Guinea pigs were employed in the test, 10 of which were treated with composition A of the invention of Examples 1, and 5 used as control. Before the test was performed, 3 additional guinea pigs were employed to conduct a preliminary test aimed to fine-tuning the sample doses to be used in the test. In fact, the test consisted of a preliminary test, an induction phase, and a stimulation phase as shown below.

Preliminary Test: A preliminary test was performed to identify a concentration of the composition to be used in the main test. For the topical induction phase, the highest concentration causing low-to-moderate erythema, without any side-effects on the animal, was selected. For the stimulation phase, the highest concentration that did not produce erythema was selected. To select the dilution of the composition sample, 4 occlusal patches were applied with 0.5 g of undiluted composition sample and diluted samples (90%, 80%, 70%, sodium chloride injection) on the back of the three additional animals. The patches were left in place for 24 hours.

Induction Phase: The guinea pigs were treated with three pairs of intradermal injections (each dose of 0.1 ml) divided as follows:
first: stable emulsion of Freud's complete adjuvant (FCA) in injection of 50:50 sodium chloride (v:v);
second: test sample for treated animals, injection of sodium chloride for control animals;
third: diluted test sample 50:50 (v:v) with stable FCA emulsion and injection of sodium chloride (50%) for treated animals, injection of 50:50 (v:v) sodium chloride with stable emulsion of FCA and injection of sodium chloride (50%) for control animals.

Six days after the intradermal injections, both for treated and control animals, an application was performed on all animals by massaging 1 ml of 10% lauryl phosphate sodium in vaseline.

Seven days after the intradermal injections, 0.5 g per animal of composition of the invention was applied on 10 animals treated for 48 hours.

The same treatment was performed on a control group using an injection of sodium chloride.

Stimulation Phase: 21 days after the treatment on all animals, both treated and control animals, the stimulation phase was performed by applying 0.5 g of the composition sample on the right side of the back, and an injection of 0.5 ml of sodium chloride on the left side. The bandage was then left for 24 hours. 48 and 72 hours after the start of the stimulation phase, the reactions both in control and treated animals were assessed.

No abnormalities were observed in animals treated with the composition sample. No abnormalities were observed in control animals.

Based on the results, that were interpreted according to the ISO standard above, the composition of the invention did not generate any sensitization.

The composition of the invention has therefore proved to be safe, well tolerated, pleasant taste, perfectly suitable for administration to adult and pediatric subjects, for the treatment of viral and bacterial gastrointestinal disorders, such as intestinal flu, food poisoning, also deriving from contaminated seafood, and traveller's diarrhea.

## Claims

1. A composition comprising diosmectite and decaffeinated green tea extract.

2. The composition according to claim 1, wherein said composition also comprises Bacillus clausii spores.

3. A formulation comprising the composition according to claim 1 or 2 and suitable excipients for oral administration.

4. The formulation according to claim 3, wherein the formulation is in the form of powder, granule, suspension, or gel.

5. The formulation according to claim 3 or 4, wherein the formulation is in the form of a single-dose sachet, vial, or stick.

6. The formulation according to any one of claims 3 to 5, comprising a unitary dose of diosmectite from 1 to 4 g, preferably 3 g.

7. The formulation according to any one of claims 3 to 6, comprising a unitary dose of green tea extract from 70 to 200 mg, preferably 100 mg.

8. The formulation according to any one of claims 3 to 7, comprising a unitary dose of Bacillus clausii from 0.01 to 1 g, preferably 0.055 g.

9. The formulation according to any one of claims 3 to 8 comprising a unitary dose of diosmectite from 1 to 4 g, a unitary dose of decaffeinated green tea extract from 70 to 200 mg, and a unitary dose of Bacillus clausii from 0.01 to 1 g.

10. The formulation according to claim 9, wherein the diosmectite unitary dose is 3 g, the decaffeinated green tea extract unitary dose is 100 mg, and the Bacillus clausii unitary dose is 0.055 g.

11. A composition according to any one of claims 1 to 2, or a formulation according to any one of claims 3 to 10, for use as a medicament.

12. A composition according to any one of claims 1 to 2, or a formulation according to any one of claims 3 to 10, for use in the treatment of viral and bacterial gastrointestinal disorders.

13. The composition or formulation for use according to claim 12, wherein gastrointestinal disorders are chosen from the group consisting of intestinal flu, food poisoning, also deriving from contaminated seafood, and traveller's diarrhea.
